Europäisches Patentamt

European Patent Office (11) Numéro de publication: **0 1 3 1 5 2 9**

Office européen des brevets **B1**

(19)

(12) **FASCICULE DE BREVET EUROPEEN**

(45) Date de publication du fascicule du brevet: **19.11.87**

(51) Int. Cl.⁴: **A 61 M 1/00**, A 61 M 1/34

(21) Numéro de dépôt: **84420117.8**

(22) Date de dépôt: **05.07.84**

(54) **Appareillage utilisable notamment en plasmaphérèse.**

(30) Priorité: **07.07.83 FR 8311572**

(43) Date de publication de la demande:
**16.01.85 Bulletin 85/3**

(45) Mention de la délivrance du brevet:
**19.11.87 Bulletin 87/47**

(84) Etats contractants désignés:
**AT BE CH DE GB IT LI LU NL SE**

(56) Documents cités:
**WO - A - 81/02979**
**DE - A - 2 745 041**

**Petit Larousse Illustré, edition 1986, p. 199**

(73) Titulaire: **RHONE-POULENC S.A., 25, quai Paul Doumer, F-92408 Courbevoie (FR)**

(72) Inventeur: **Malbrancq, Jean-Michel, 7, rue Jeanne d'Arc, F-94320 Thiais (FR)**
Inventeur: **Vantard, Georges, 6, impasse du Rafo, F--38290 Villefontaine (FR)**

(74) Mandataire: **Vogt, Bernard et al, RHONE-POULENC INTERSERVICES Service Brevets Chimie Centre de Recherches de Saint-Fons B.P. 62, F-69192 Saint-Fons Cédex (FR)**

## Description

La plasmaphérèse est une technique consistant à séparer le sang entier d'un donneur en deux parties, la première partie constituant la phase plasmatique, tandis que la seconde partie constitue la phase cellulaire qui est en général réinjectée au donneur. La phase plasmatique est une solution aqueuse complexe contenant notamment des protéines, tandis que la phase cellulaire contenant encore du plasma comprend les globules rouges (ou hématies), les globules blanc (ou leucocytes) et les plaquettes.

La technique de plasmaphérèse est utilisée depuis fort longtemps en expérimentation animale. A titre indicatif on peut citer l'article de John J. ABEL et al. ayant pour titre «Plasma removal with return of corpuscules», paru dans J. Pharmacol. Exp. Ther. en 1914, n° 5, pages 625 à 641 dans lequel du sang de chien est centrifugé pour faire la séparation. On peut citer également l'article de A. GEIGER paru en 1931 dans J. Phys. 71, pages 111–120 ayant pour titre «Method of Ultrafiltration in vivo» dans lequel il est décrit une opération de plasmaphérèse en continu sur chien, l'appareil de séparation utilisé étant un séparateur à membrane, cette dernière étant disposée en spirale et étant choisie de façon telle qu'il soit possible d'obtenir une solution plasmatique comprenant la totalité des protéines du sang traité, lorsque cela est désiré.

La plasmaphérèse est également appliquée à l'homme depuis un certain nombre d'années comme l'indique l'article «La plasmaphérèse – Technique – Indications» de Fr. OBERLING et al. paru dans J. Med. Strasbourg 1968, mars, pages 277–279. La plasmaphérèse tend ainsi maintenant à supplanter le don total du sang car cette technique présente l'avantage de permettre de prélever à l'homme de plus grandes quantités de plasma sans inconvénient. Du fait que l'on restitue au donneur les éléments figurés du sang, les séances de prélèvement peuvent être plus rapprochées dans le temps que pour le don du sang.

Ainsi la plasmaphérèse est une technique ancienne et les perfectionnements ultérieurs lui ayant été apportés portent soit sur des appareils à centrifugation, soit sur des appareils à membranes. Parmi les brevets de perfectionnement concernant des appareils à membranes, on peut citer DE-A-2 100 209 de AMICON dans lequel il est décrit un récipient comprenant une membrane formant une spirale pour la circulation du sang entier prélevé d'un donneur et dans lequel on exerce une pression sur le sang contenu dans le récipient, soit au moyen d'un gaz, soit au moyen du piston d'une seringue soumis à l'action d'un ressort à lame. Par rapport à l'appareil de GEIGER précédemment décrit, cet appareil de par sa conception présente l'inconvénient de ne pas permettre d'opérer en continu sur le donneur. On peut citer également US-A-4 191 182 de HEMO-THERAPY Inc., dans lequel il est décrit un appareillage à membrane et dans lequel le sang prélevé en continu au donneur est séparé en plasma et en une fraction cellulaire retournant en continu au donneur, cet appareillage ayant notamment pour caractéristique le fait de permettre à une partie de la fraction cellulaire de recirculer dans le compartiment amont de l'appareil à membrane et le fait de permettre à la fraction plasmatique de recirculer dans le compartiment aval du même appareil. On peut citer de plus la demande internationale déposée par FRIEDMAN et al. et publiée sous le n° WO 79/01 121, dans laquelle il est également question d'un appareillage permettant de retirer le sang du donneur et de lui réinjecter la fraction n'ayant pas traversé la membrane, de façon continue. Il peut être cité en outre la demande internationale WO 81/02 979 se rapportant à un appareil pour le fractionnement du sang avec lequel le sujet est relié par deux aiguilles.

Cependant les appareillages décrits ci-avant et permettant la plasmaphérèse en continu ont notamment pour inconvénient d'exiger de piquer le donneur en deux endroits distincts, ce qui est assez désagréable pour celui-ci.

Un but de la présente invention est donc un appareillage utilisant un appareil à membrane et permettant notamment des opérations de plasmaphérèse sur un donneur en ne le piquant qu'à un seul endroit avec une aiguille classique de transfusion sanguine.

Un autre but de la présente invention est un appareillage permettant, au cours d'une séance, de faire circuler plusieurs fois le sang en provenance du donneur jusqu'à une poche à sang, puis de la poche à sang au donneur, tandis que le sang passe en continu et dans le même sens de circulation au contact d'un appareil séparateur à membrane.

Un autre but de la présente invention est un appareillage spécialement adapté permettant d'obtenir un plasma de très bonne qualité et dans les meilleures conditions de rendement de filtration, tout en assurant une hémolyse pratiquement nulle du sang.

Un autre but de la présente invention est un appareillage permettant de réguler la pression de sortie de la fraction cellulaire du compartiment amont de l'appareil à membrane à des valeurs comprises généralement entre 0 et 20 mm de mercure en pression relative, le compartiment aval étant à la pression atmosphérique.

Un autre but de la présente invention est un appareillage permettant de retirer d'un donneur environ 600 ml de plasma en environ 45 minutes et même en moins de temps.

Un autre but de la présente invention est un appareillage de prélèvement de plasma dans lesquels il est possible d'adapter facilement la stratégie opératoire en fonction du donneur, des désirs de l'opérateur et des caractéristiques de l'appareil à membrane utilisée.

Un autre but de la présente invention est un appareillage permettant d'obtenir un rendement de séance supérieur au rendement intrinsèque de l'appareil à membrane. On entend par rendement de séance le rapport entre le débit de liquide qui a traversé la membrane (c'est-à-dire le plasma) et le débit de sang prélevé ou restitué à la veine du donneur. On entend par rendement intrinsèque de l'appareil à membrane le rapport du débit de plasma filtré au débit du sang à l'entrée de l'appareil à membrane.

Un autre but de la présente invention est un appareillage permettant de faire passer le sang au contact de la membrane à un débit supérieur à celui du sang prélevé au donneur.

Un autre but de la présente invention est un appareillage dans lequel les volumes extracorporels de sang mis en circulation sont faibles.

Un autre but de la présente invention est un appareillage se prêtant également bien à la plasmaphérèse avec restitution au patient, lors de la phase de réinjection (ou de retour) d'un liquide de remplacement dont le volume correspond à celui du plasma retiré.

De façon plus générale un but de la présente invention est un appareillage permettant de prélever sur un sujet, homme ou animal, en ne le piquant qu'une seule fois, un liquide que l'on amène au contact de la (ou des) membrane(s) d'un appareil à membrane(s) semi-perméable(s) pendant une phase dite de prélèvement, ledit liquide passant au contact de la membrane étant séparé en une fraction traversant la membrane et en une fraction ne traversant pas la membrane, puis de faire retourner au sujet, lors de la phase dite de retour, la fraction du liquide prélevé n'ayant pas traversé la membrane, le liquide au contact de la membrane circulant dans le même sens au cours d'une séance qui peut comporter plusieurs phases de prélèvement et de retour.

Il a maintenant été trouvé, et c'est ce qui fait l'objet de la présente invention, un appareillage utilisable dans le domaine médical, notamment en plasmaphérèse comprenant en combinaison:
— un dispositif (1) de prélèvement d'un liquide d'un sujet,
— un appareil (2) à membrane semi-perméable séparant ce liquide en une fraction ayant traversé la membrane et en une fraction n'ayant pas traversé la membrane, ledit appareil comportant un compartiment amont (3) et un compartiment aval (4) séparés par une membrane (14),
— une canalisation (5) reliant le dispositif (1) de prélèvement à une tubulure (6) du compartiment amont (3) de l'appareil (2) à membrane,
— une pompe (7) disposée sur cette canalisation (5),
— un capteur de pression (13) du liquide circulant dans la canalisation (5), ledit capteur étant situé entre le dispositif (1) de prélèvement et la pompe (7), ce capteur agissant sur la vitesse de rotation de la pompe (7) qui lui est asservie,
— un récipient (20) de recueil du liquide ayant traversé la membrane, ce récipient (20) étant en communication avec la sortie du compartiment aval (4) de l'appareil (2) à membrane,
— des moyens (19) permettant de s'assurer que la pression transmembranaire du liquide circulant au contact de la membrane (14) ne dépasse pas une valeur de consigne, caractérisé en ce qu'il comprend:

— une canalisation (16) reliant une tubulure (15) du compartiment amont (3) de l'appareil (2) à membrane à un récipient (17) de recueil de la fraction du liquide n'ayant pas traversé la membrane,
— une canalisation (16b) reliant le récipient (17) à la canalisation (5), la jonction entre la canalisation (16b) et la canalisation (5) se faisant en un point A situé entre la pompe (7) et la tubulure (6) du compartiment amont (3) de l'appareil (2) à membrane semi-perméable,
— une pompe (18) disposée sur la boucle définie par la canalisation (16), la canalisation (16b) et la partie de la canalisation (5) comprise entre la jonction A et la tubulure (6) du compartiment amont (3) de l'appareil (2) à membrane, les moyens (19) agissant sur la vitesse de rotation de la pompe (18) qui leur est asservie,
— en ce que la pompe (7) est capable de tourner dans les deux sens et en ce que le dispositif (1) de prélèvement sert à la réinjection au sujet de la fraction n'ayant pas traversé la membrane, ledit dispositif étant constitué d'une seule aiguille.

La description de la présente invention sera mieux comprise à l'aide des figures ci-jointes, qui illustrent, de façon schématique, à titre d'exemples non limitatifs et sans échelle déterminée, des modes de réalisation particuliers dudit appareillage.

La figure 1 représente un mode de réalisation de l'appareillage selon la présente invention.

La figure 4 représente l'appareillage selon la figure 1 dans lequel ont été notamment ajoutées les connexions à un dispositif de contrôle et de commande.

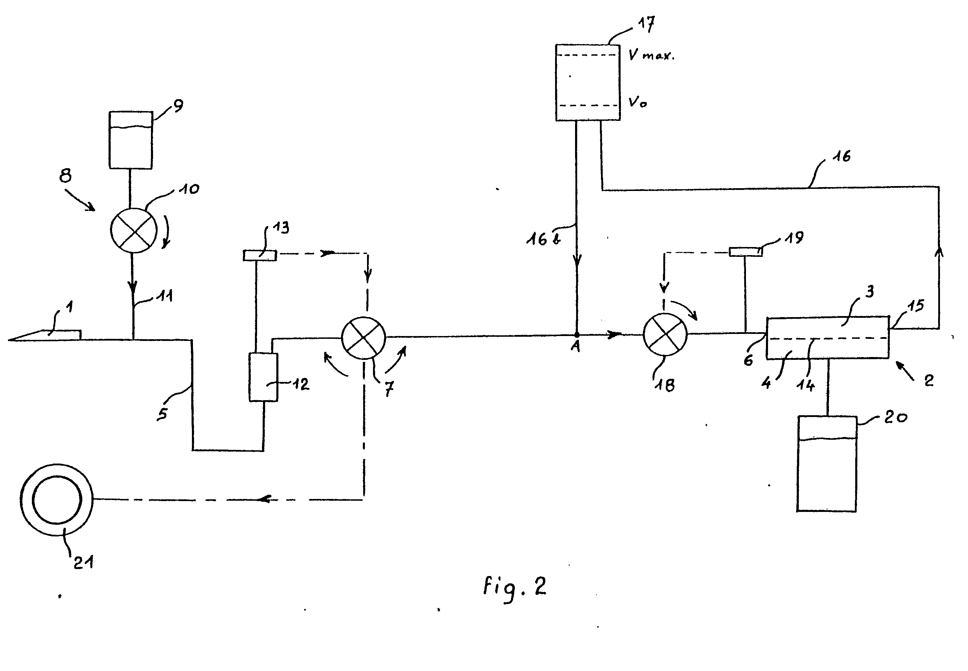

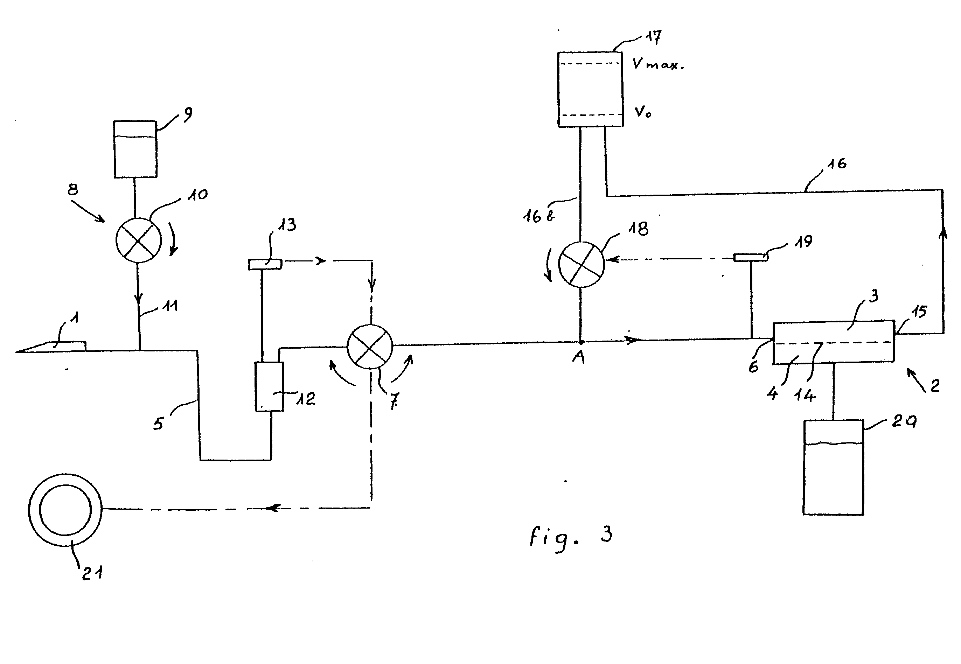

Les figures 2 et 3 représentent deux autres modes de réalisation d'un appareillage selon la présente invention.

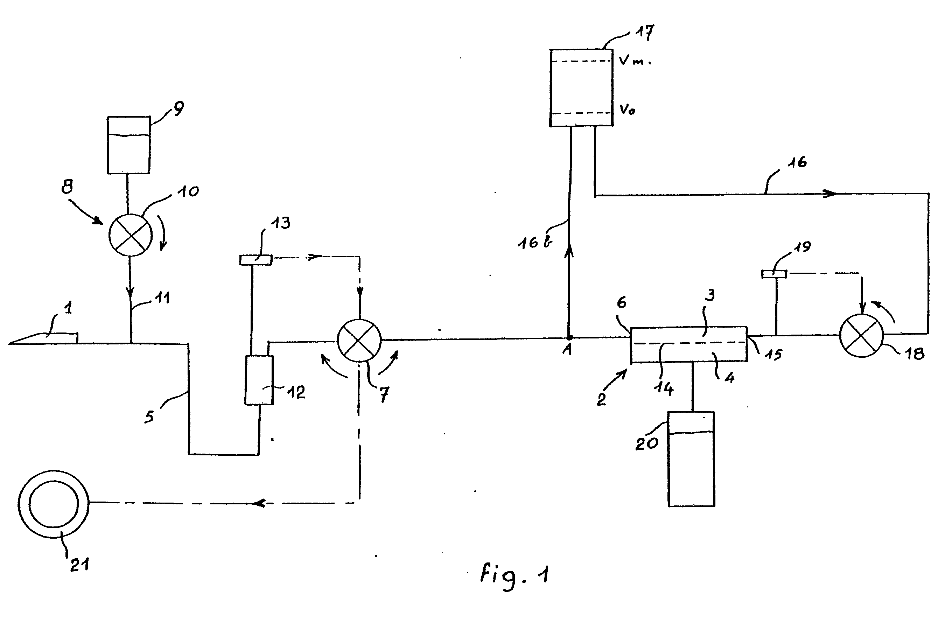

L'appareillage selon la figure 1, plus spécialement adapté à la plasmaphérèse de don, comprend un dispositif (1) de prélèvement du sang du donneur, constitué avantageusement d'une aiguille de prise de sang. A titre indicatif cette aiguille peut avoir un diamètre externe de 1,65 mm et un diamètre interne de 1,45 mm, telle que celles répertoriées dans les centres de transfusion sanguine sous le vocable 16 G. Un appareil (2) à membrane (14) semi-perméable, comportant un compartiment amont (3) et un compartiment aval (4) est relié à l'aiguille (1) par une canalisation (5) allant de l'aiguille (1) à une tubulure (6) en communication avec le compartiment amont (3) de l'appareil à membrane. Cette canalisation (5) est généralement constituée par un tube en matière plastique, par exemple en polychlorure de vinyle. Sur cette canalisation ou ligne (5) se trouve une pompe (7) pouvant tourner dans les deux sens, avantageusement du genre pompe péristaltique. Entre la pompe (7) et l'aiguille (1) est disposé un dispositif (8) destiné à transmettre un produit anticoagulant au sang en provenance du donneur, par exemple une solution glucosée contenant 35,6 g/l de citrate trisodique, référence AB 16 de la Société BIELUZ. Ce dispositif (8) comprend par exemple un réservoir (9) d'anticoagulant, une canalisation (11) raccordée à la canalisation (5) et au réservoir (9), et une pompe (10) par exemple péristaltique, située sur la canalisation (11). Cette canalisation (11) se raccorde sur la canalisation (5) le plus près possible de l'aiguille (1). Entre le point de raccordement des canalisations (11 et 5) et la pompe (7), un détecteur (12) de bulles et un capteur (13) de pression sont avantageusement sur la canalisation (5). La tubulure (15) du compartiment amont (3) de l'appareil (2) à membrane est reliée par une canalisation (16) à un récipient (17), ou poche, de recueil du sang, ce récipient (17) étant lui-même relié par la canalisation (16b) à la canalisation (5), la jonction entre la canalisation (5) et la canalisation (16b) se faisant en un point A situé entre la pompe (7) et la tubulure (6) de l'appareil (2) à membrane. Pour la clarté de la description ce point A sera appelé parfois jonction A. Les canalisations (16 et 16b) peuvent être en même matière et aux mêmes diamètres que la canalisation (5), tandis que le récipient (17) peut être constitué par une poche en matière plastique souple. Les canalisations (16, 16b) et la partie de la canalisation (5) située entre la jonction A et la tubulure (6) de l'appareil (2) à membrane constituent une boucle de circulation de la fraction du sang ne traversant pas la membrane (14) et passant dans le compartiment amont (3) ainsi que dans le récipient (17). Le compartiment aval (4) de l'appareil (2) à membrane est relié à un récipient (20) de recueil du plasma ayant traversé la membrane (14), ce récipient (20) étant par exemple une poche en matière plastique.

Dans la boucle définie ci-avant se trouvent de plus un capteur de pression (19) et une pompe (18) de recirculation du sang.

Dans l'appareillage selon la figure 1 la pompe (18) est située, dans la bouche précédemment définie, sur la canalisation (16) à proximité de la tubulure (15) de l'appareil séparateur tandis que le capteur de pression (19) est situé entre ladite tubulure (15) et la pompe (18).

Dans l'appareillage selon la figure 2, la pompe (18) est située, dans la boucle précédemment définie, entre le point de jonction A et la tubulure (6) de l'appareil (2) à membrane, le capteur de pression (19) étant positionné entre la pompe (18) et ladite tubulure (6).

Dans l'appareillage selon la figure 3 la pompe (18) est située sur la canalisation (16b) tandis que le capteur de pression (19) est sur la canalisation (5), entre le point de jonction A et la tubulure (6) de l'appareil (2) à membrane.

Dans l'appareillage selon les figures 1, 2 et 3 précédemment décrites, le capteur (19) de pression agit sur la vitesse de rotation de la pompe (18) qui lui est asservie. Les flèches situées sur les canalisations (16b et 16) indiquent le sens de recirculation du sang dans la boucle au cours d'une séance de don de plasma. Le capteur (19) permet de s'assurer que le sang ne circule pas à une pression supérieure à une valeur de consigne choisie, c'est-à-dire que la pression transmembranaire dans l'appareil (2) à membrane ne s'élève pas en-dessus d'une valeur de consigne choisie, par exemple pour que le sang ne s'hémolyse pas.

L'appareillage représenté figures 1 à 4 comprend de plus un garrot (21) connu en soi, ledit garrot pouvant être gonflé et dégonflé lorsque cela est désiré, par un dispositif connu en soi, par exemple par un compresseur, un capteur de pression arrêtant le gonflement du garrot (21) lorsqu'une pression de consigne est atteinte. La ligne représentée en trait mixte sur les figures 1 à 3, entre le garrot (21) et la pompe (7), montre que le gonflement ou dégonflement du garrot (21) dépend du sens de rotation de la pompe (7). Lors de la phase de prélèvement du sang du donneur le garrot (21) est gonflé, tandis que lors de la phase de retour au donneur, du sang n'ayant pas traversé la membrane, le garrot (21) est dégonflé.

L'appareil (2) dont il a été question ci-avant peut avoir sa membrane sous forme plane, sous forme spiralée, ou sous forme de petits tubes fins tels que des fibres creuses. Lorsque la membrane comprend une pluralité de fibres creuses, le sang circule avantageusement à l'intérieur des fibres creuses, l'ensemble des parties internes des fibres constituant le compartiment (3) amont de l'appareil. Lorsque la membrane est sous forme plane ou spiralée, le sang circule avantageusement entre deux membranes ou des groupes de deux membranes qui constituent le compartiment amont (3) de l'appareil (2) à membrane.

Les membranes utilisées pour des opérations de plasmaphérèse sont de préférence celles qui permettent de recueillir un plasma : – dans lequel toutes les protéines du sang initial sont retrouvées dans les mêmes proportions, – dont la concentration protéique est supérieure à 55,5 g/l, – dans lequel il n'y a pas de globules rouges et dans lequel la concentration plaquettaire est inférieure à 15 000 plaquettes par $mm^3$. Les membranes choisies sont celles permettant également de ne pas hémolyser le sang circulant à leur contact, tout en offrant de bons rendements de filtration.

Ces membranes de plasmaphérèse ont avantageusement un taux de rejet au latex, dont les particules sont calibrées à 0,27 microns, inférieur à 75% et elles ont un taux de rejet au latex, dont les particules sont calibrées à 0,64 microns, supérieur à 15%. Préférentiellement le taux de rejet aux particules calibrées à 0,27 microns est inférieur à 30% et le taux de rejet aux particules de latex calibrées à 0,64 microns est supérieur à 90%.

Pour effectuer cette mesure de taux de rejet au latex, on opère de la façon suivante, lorsque les membranes sont planes.

Dans une cellule de type AMICON Modèle 52, on charge 50 ml de suspension de particules calibrées de polystyrène de diamètre 0,27–0,4 ou 0,64 microns (vendues par la Société RHONE-POULENC sous la marque ESTAPOR) diluée à 0,1% avec de l'eau distillée additionnée de 1% d'agent tensio-actif (alkylarylsulfonate marque SINOZON NAS 60, fabriqué par la Société SINNOVA).

La cellule AMICON est équipée d'un échantillon de la membrane tramée. On établit une pression d'air correspondant à 20 cm d'eau. On récupère les six premiers millilitres de filtrat dont on détermine la concentration (cf) en particules calibrées.

Le taux de rejet est défini par la formule:

$$\frac{(0{,}1 - cf) \times 100}{0{,}1}$$

Des membranes ayant les caractéristiques ci-avant sont généralement en matériau synthétique, par exemple en esters de cellulose nitrate de cellulose ...), en cellulose régénérée, en polycarbonate ... Ces membranes peuvent être également à base de polyétheruréthanes comprenant des groupements ammonium héparinés, ou en copolymère d'acrylonitrile. De façon avantageuse ces membranes sont renforcées par une trame lorsqu'elles sont sous forme de membranes planes et ont une épaisseur comprise entre 50 et 200 microns.

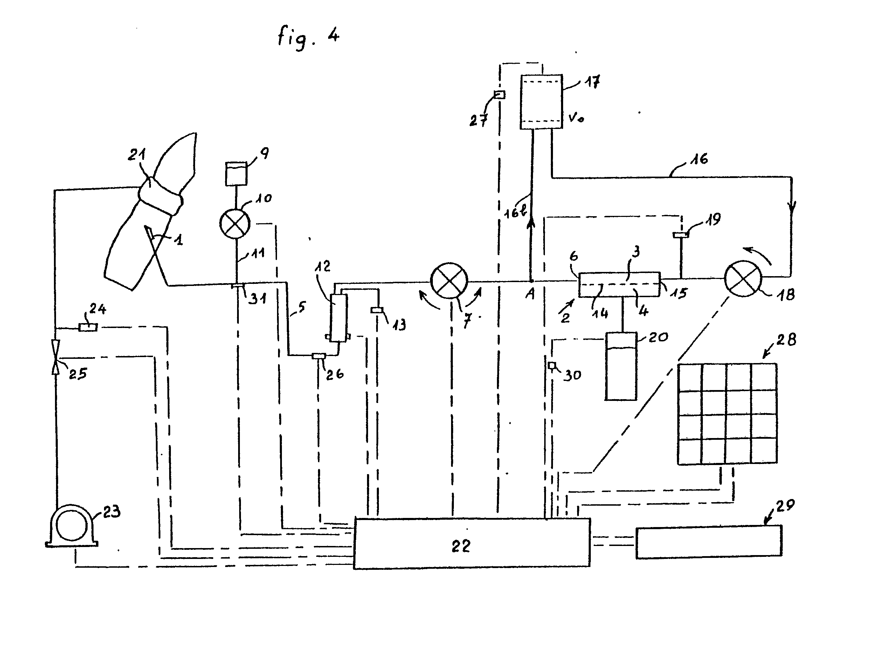

La figure 4 représente un appareillage équivalent à celui selon la figure 1, mais dans lequel on trouve les connexions électriques des différents éléments à une unité logique (22) de commande et de contrôle, les lignes électriques étant représentées en traits mixtes. L'unité (22) est reliée à une source de courant (non représentée). Bien entendu tous les éléments de l'appareillage peuvent être groupés sur une console ou pupitre comprenant par exemple des roulettes, pour mieux la déplacer. La description de l'unité logique (22) ainsi que du clavier (28) et de l'afficheur (29) dont il sera question ci-après n'a pas été développée car la réalisation des circuits électroniques et l'utilisation avantageuse de microprocesseurs et de mémoires est évidente pour le technicien considéré une fois le problème posé, c'est-à-dire après qu'on lui ait demandé de faire en sorte que l'appareillage décrit puisse fonctionner de façon automatique et fiable. Les appareillages selon les figures 2 et 3 sont avantageusement, et de la même façon, reliés à une unité (22) logique de commande et de contrôle non représentée.

Pour la mise en œuvre de l'appareillage selon les figures 1 et 4 on opère de la façon suivante, par exemple dans le cas d'une opération de plasmaphérèse de don. On commence par remplir la canalisation (11) de solution citratée et comme la jonction de la canalisation (11) avec la canalisation (5) est en fait très proche de l'aiguille (1), on peut considérer que l'aiguille (1) est au moins en partie remplie de cette solution citratée. Le garrot ayant été préalablement gonflé à la pression souhaitée (aux environs de 60 mm de mercure) grâce au compresseur (23) par action de l'électrovanne (25) et du manomètre à contact (24), on enfonce l'aiguille (1) dans une veine du donneur après avoir préparé classiquement l'endroit de piqure, situé entre le garrot et l'extrémité du membre. A ce moment la pompe (10) envoie du citrate dans la canalisation (5), tandis que la pompe (7) tourne dans le sens qui fait passer le sang vers la tubulure (6) de l'appareil (2) à membrane et dans le récipient (17) par la canalisation (16b). La pompe (7) est asservie au capteur (13) de pression de façon à ce que la pression mesurée à cet endroit de la ligne (5) reste toujours supérieure à une certaine valeur, généralement proche de 0 mm de mercure, appelée pression de seuil, pour s'assurer que la pompe (7) ne tire pas le sang directement de la veine du donneur. Si la pression dans cette partie de la ligne devient inférieure à la pression de consigne fixée au capteur (13), automatiquement l'unité logique (22) agit et arrête ou ralentit momentanément la rotation de la pompe (7), tant que la pression voulue n'est pas revenue. Lorsque le sang dans le récipient (17) atteint un volume Vo minimum, la pompe (18) se met en marche et fait circuler le sang au contact de la membrane (14) dans le compartiment amont (3) de l'appareil (2), le sang suivant le trajet indiqué par les flèches dans la boucle contenant les canalisations (16b et 16). La pompe (18) associée au capteur de pression (19) est placée dans la boucle (16, 16b) de façon à être en amont de l'appareil à membrane (2). Le capteur (19) indique la pression d'entrée du sang dans l'appareil à membrane (2). La détermination du volume Vo peut être faite par un dispositif tachymétrique en relation avec la pompe (7). La pompe (18) peut pendant un temps très court (par exemple 15 secondes) tourner à la même vitesse que la pompe (7), puis ensuite sa vitesse de rotation augmente de façon à ce que la vitesse de circulation du sang dans la boucle soit supérieure à celle du sang dans la canalisation (5) en dehors de la boucle. La vitesse de circulation du sang dans la boucle peut par exemple être 1,5 à 7 fois supérieure à celle du sang prélevé au donneur. Le capteur de pression (19) permet de s'assurer que la pression transmembranaire dans l'appareil (2) à membrane ne dépasse pas une valeur de consigne prévue, pour ne pas hémolyser le sang. Le capteur (19) de pression est réglé de façon à ce que le sang pénètre dans le compartiment amont (3) de l'appareil (2) à membrane à une pression généralement comprise entre 40 et 100 mm de mercure en valeur relative, de préférence entre 50 et 90 mm de mercure, lorsque le plasma est à la pression atmosphérique dans le compartiment (4) aval de l'appareil (2) à membrane. Si cette valeur maximale souhaitée est dépassée, automatiquement l'unité logique (22) arrête ou ralentit la pompe (18).

La période pendant laquelle le sang sort de la veine du donneur est appelée phase de prélèvement. Celle-ci se termine par exemple en fonction du volume prédéterminé de sang que l'on veut prélever au donneur (à chaque phase de prélèvement), ce volume étant bien entendu toujours inférieur au volume du récipient (17) de recueil du sang en circulation dans la boucle. De façon avantageuse un dispositif tachymétrique est branché sur la pompe (7) et lorsque le volume souhaité de sang est branché sur la pompe (7) et lorsque le volume souhaité de sang est prélevé au donneur, l'unité logique (22) agit et arrête les pompes (7 et 10). Au cours de cette phase de prélèvement le sang circulant dans la boucle a tendance à voir son hématocrite augmenter; le capteur (19) agira pour ralentir la vitesse de la pompe (18) lorsque la pression augmentera. L'unité logique (22) agit sur l'électro-vanne (25) qui se positionne de façon telle que le garrot (21) se dégonfle et elle met en marche la pompe (7) dans le sens inverse de celui de la phase précédente, dite de prélèvement. Alors commence la phase dite de retour, au cours de laquelle le sang contenu dans la poche (17) retourne au donneur tout en circulant en partie dans la boucle (16, 16b) en passant au contact de la membrane (14) de l'appareil (2). Au cours de cette phase de retour comme précédemment lors de la phase de prélèvement le sang circulant dans la boucle a tendance à voir son hématocrite augmenter, car du plasma passe encore dans le récipient (20). Le capteur (19) agira dans ce cas pour ralentir la vitesse de rotation de la pompe (18) lorsque la pression augmentera. On peut prévoir d'ailleurs, dès le commencement de la phase de retour, de ralentir quelque peu la vitesse de rotation de la pompe (18), tout en lui faisant circuler le sang sans la boucle à une vitesse supérieure à celle du sang retournant au donneur. Au cours de la phase de retour, pendant laquelle la pompe (10) citrate est arrêtée, le sang passe dans le détecteur (12) de bulles. Si des bulles sont détectées par ce dernier (12), l'unité logique (22) arrête immédiatement la pompe (7) et éventuellement agit sur un «clamp» (26), ou dispositif d'obturation, qui ferme la canalisation (5). Au cours de la phase de retour le capteur (13) de pression sert de sécurité en ce sens qu'il est réglé de façon telle que la pression du sang ne dépasse pas une certaine valeur prédéterminée. Si cette valeur est dépassée, par exemple par obstruction totale ou partielle de l'aiguille (1), l'unité logique (22) intervient immédiatement pour arrêter ou ralentir la pompe (7). Au cours de la phase de retour le sang peut éventuellement passer au travers d'un filtre classique prévu dans le détecteur de bulles pour éviter que des particules indésirables puissent être renvoyées au donneur. Ce filtre peut par exemple s'affacer lors de la phase de prélèvement, tandis qu'il se rabat sur un siège, prévu dans le détecteur de bulles, lors de la phase de retour. Pendant la phase de retour la pompe (18) tourne dans le même sens de rotation que celui qu'elle avait lors de la phase de prélèvement, le sang circulant ainsi dans la bouche (16, 16b) selon le sens des flèches pendant les phases de prélèvement et de retour. La fin de la phase de retour est détectée par exemple par un dispositif (27) à peson relié à l'unité logique (22), ledit peson permettant d'arrêter la phase de retour lorsque le volume Vo est atteint dans le récipient (17). L'unité logique (22) fait alors fonctionner à nouveau l'appareillage en phase de prélèvement, c'est-à-dire que le garrot (21) est gonflé après que le compresseur (23) se soit mis en marche et que la vanne (25) se soit positionnée en position adéquate, et met en marche la pompe (7) en sens inverse pour que le sang du donneur aille de l'aiguille (1) vers le récipient (17) dans la boucle comprenant notamment les canalisation (16) et (16b) ainsi que le compartiment (3) amont de l'appareil (2) à membrane. La pompe (10) de distribution de produit anticoagulant est également mise en marche dès le début de la phase de prélèvement. Lorsqu'au cours de la dernière phase de retour on s'aperçoit que le récipient (20) de recueil de plasma est suffisamment rempli, on arrête complètement l'opération après avoir vidé le récipient (17) et la canalisation (5) au moins jusqu'au détecteur (12) de bulles au cours de la dernière phase de retour.

Généralement le débit de la pompe (10) est réglé de façon que, lors de la phase dite de prélèvement, on ait un volume de citrate pour 8 volumes de sang ou de préférence 1 volume de citrate pour 16 volumes de sang, le rapport étant choisi par l'utilisateur de l'appareillage. Ce rapport de dilution est avantageusement obtenu en asservissant la vitesse de rotation de la pompe (10) à celle de la pompe (7).

Il apparaît clairement que l'appareillage selon la présente invention peut faire l'objet d'une automatisation très poussée. Ainsi comme celà est représenté plus particulièrement sur la figure 4, l'unité logique (22) de commande et de contrôle peut être reliée à un clavier (28) et à un afficheur (29). De même l'unite logique (22) peut être reliée à un tableau synoptique (non représenté) sur lequel la localisation de toute anomalie est signalée par un voyant lumineux à l'opérateur, en même temps par exemple qu'un signal sonore est mis en route. Sur le clavier (28) à touches on peut choisir le volume de sang maximum que l'on veut faire circuler pendant la phase de prélèvement (300, 350, 400, 450 cm³ de sang par exemple) en appuyant sur la touche correspondante. On peut également choisir le volume de plasma que l'on veut prélever durant la séance (400, 500 ou 600 cm³ par exemple) en appuyant sur la touche correspondante. Ainsi un dispositif (30) est avantageusement prévu sur la poche (20) de plasma pour connaître instantanément la quantité de plasma prélevé en cours de séance, ce dispositif (30) connu en soi étant connecté à l'unité logique (22). Sur le clavier (28) peut également être prévue une touche pour l'amorçage automatique de la ligne (11) avant de piquer le donneur. En appuyant sur cette touche la pompe (10) se met en route et s'arrête automatiquement lorsque la solution de citrate est décelée par exemple à la jonction (31) des deux lignes (11 et 5). Sur le clavier (28) on peut également prévoir par exemple une touche pour connaître sur l'afficheur (29) le volume instantané de plasma dans la poche (20) à tout moment, une touche pour connaître sur l'afficheur (29) le débit du sang de la pompe (7), pour connaître le temps de séance en cours ... L'appareillage peut comprendre, en liaison avec l'unité logique (22) et les valeurs affichées au clavier (28) en ce qui concerne le volume de sang souhaité pendant la phase de prélèvement et le volume total de plasma désiré, un système intégrateur agissant au cours de la dernière phase de prélèvement pour que le volume de prélèvement permette d'obtenir le volume total souhaité de plasma à la fin de la dernière phase de retour.

De nombreuses variations de l'appareillage précédemment décrit apparaîtront au technicien. A titre d'exemple l'appareillage peut comporter un ballonnet collabable sur la ligne (5) entre la jonction (31) et le clamp (26). Ce ballonnet sert alors de double sécurité avec le capteur de pression (13) en ce sens qu'il se ferme lorsque le débit de la pompe (7) est supérieur à celui de la veine, si le capteur n'a pas fonctionné au cours de la phase de prélèvement. Eventuellement ce ballonnet collabable peut être substitué au capteur (13).

De même le dispositif (8) destiné à transmettre le produit anticoagulant peut éventuellement être omis dans la mesure où l'intérieur de l'aiguille (1), du détecteur de bulles (12) et des lignes (5, 16 et 16b) est par exemple recouvert d'un polymère à base de polyétheruréthanes à groupes ammonium héparinés, tels ceux décrits notamment dans le brevet américain 4 046 725. Eventuellement les lignes (5 et 16) peuvent être en un polymère tel que ceux décrits dans le brevet américain cité ci-avant ou en un mélange de polychlorure de vinyle et de polyétheruréthane à groupes ammonium héparinés tel que ceux des mélanges dont il est question dans la demande de brevet européen 12 701. La membrane microporeuse peut elle aussi être préparée à partir d'un mélange de polymère selon la demande de brevet européen 12 701. De même l'appareillage peut comporter un détecteur connu en soi sur la ligne (11) permettant de signaler l'absence d'anticoagulant dans le réservoir (9). De même l'appareillage peut comporter un détecteur optique sur la ligne reliant le récipient (20) à l'appareil à membrane (2) permettant de signaler la présence d'hémoglobine dans le plasma filtré.

Les appareillages selon les figures 2 et 3 comprenant les mêmes éléments constitutifs que celui selon la figure 1 ou 2, avec toutefois pour différence le fait que la pompe (18) et le capteur (19) de pression sont positionnés d'une autre façon dans la boucle comprenant les canalisations (16 et 16b). Dans le cas de l'appareillage selon la figure 2, la pompe (18) se trouve sur la portion de la canalisation (5) comprise entre le point de jonction A et la tubulure (6) du compartiment amont (3) de l'appareil (2), le capteur (19) étant positionné entre la pompe (18) et ladite tubulure (6). Dans le cas de l'appareillage selon la figure 3, la pompe (18) se trouve sur la canalisation (16b), tandis que le capteur (19) se trouve à proximité de la tubulure (6) du compartiment (3) amont de l'appareil (2), sur la portion de la canalisation (5) comprise entre le point de jonction A et la tubulure (6). Dans les appareillages selon les figures 2 et 3, le sens de rotation de la pompe (18) est tel que celle-ci et le capteur (19) qui lui est associé se trouvent, comme dans l'appareillage selon la figure 1, en amont de l'appareil à membrane (2).

Pour la mise en œuvre de l'appareillage selon la figure 2, lors du commencement de la première phase de prélèvement au donneur, on peut faire tourner la pompe (18) à la même vitesse que celle de la pompe (7), tant que le volume Vo n'est pas atteint dansle récipient (17), puis on accélère la vitesse de rotation de la pompe (18). Lors d'une séance de plasmaphérèse le sang circule selon le sens des flèches dans la boucle, c'est-à-dire en passant du compartiment (3) amont de l'appareil (2) dans la canalisation (16), puis dans le récipient (17), la tubulure (16b) et retourne dans le compartiment (3) amont de l'appareil (2).

Pour la mise en œuvre de l'appareillage selon la figure 3, lors du commencement de la première phase de prélèvement à partir du donneur, on peut commencer par faire tourner la pompe (18) de façon à ce qu'elle amène dans le récipient (17) une fraction du sang prélevé, tandis que l'autre fraction du sang prélevé passe dans le compartiment (3) amont de l'appareil (2) à membrane en entrant par la canalisation (6) et va ensuite dans le récipient (17). Lorsque le volume Vo est atteint dans le récipient (17) le sens de rotation de la pompe (18) est alors inversé et elle se met à tourner à une vitesse permettant d'avoir une vitesse de circulation du sang dans la boucle plus élevée que la vitesse de circulation du sang prélevé au donneur. Au cours de la séance, dans la boucle, le sang circule en suivant le même sans de circulation que celui selon l'appareillage de la figure 2, c'est-à-dire qu'il passe du compartiment (3) amont de l'appareil (2) à membrane dans la canalisation (16), puis dans le récipient (17) et dans la canalisation (16b) pour retourner à la tubulure (6) du compartiment (3) amont de l'appareil (2) à membrane.

Avec l'appareillage tel que représenté figures 1 et 4 et précédemment décrit, des opérations de plasmaphérèse ont été effectuées sur un donneur en utilisant à titre d'exemple un appareil (2) à membrane dont la surface membranaire totale est de 600 cm$^2$ et comprend deux membranes disposées face à face constituant le compartiment amont (3) entre lesquelles le sang circule. Les membranes ont chacune 30 cm de longueur et 10 cm de largeur et sont tramées, comme ceci est mieux décrit ci-après. L'épaisseur moyenne du film sang est de 500 microns. Le dispositif (1) de prélèvement est une aiguille de 1,65 mm de diamètre externe et de 1,45 mm de diamètre intérieur. Les canalisations (5, 16 et 16b) sont en polychlorure de vinyle (PVC) et on un diamètre intérieur de 3,5 mm. La canalisation (11) est en PVC et a un diamètre interne de 0,9 mm. La pompe (10) est une pompe péristaltique (référence RP 04 commercialisée par la Société HOSPAL), ladite pompe ayant un corps de pompe en silicone.

Les pompes (7 et 18) sont des pompes péristaltiques (référence RP 01, commercialisées par la Société HOSPAL), lesdites pompes ayant un corps de pompe en silicone. Les récipients (17 et 18) ont une contenance de 1000 cm$^3$ et sont en PVC.

La membrane utlisée est une membrane tramée obtenue à partir d'une solution dans un solvant organique de polymère que l'on coule sur une trame tournant au contact d'une bande dont la surface est très lisse. Cette solution comprend 8% en poids dans un mélange N-méthylpyrolidone/glycérine (70,8/21,2%) d'un copolymère d'acrylonitrile/méthacrylate de méthyle/méthallyl sulfonate de sodium, comprenant 7,75% en poids de méthacrylate et 80 mEg/kg de sites acides. Ce copolymère a une viscosité spécifique de 0,3 à 20 °C en solution à 2 g/l dans le diméthylformamide.

La trame utilisée est un tissu monofilament en polytéréphtalate d'éthylène-glycol, dont la maille est de 75 microns, le diamètre du fil est de 55 microns et le taux de vide est de 33%. Le grammage de cette trame est de 55 g/m².

La membrane tramée microporeuse obtenue a une épaisseur de 120 microns et son grammage est de 10 g de polymère par m² de membrane sèche.

La microstructure de la phase polymère de la membrane est spongieuse et régulière. Sa porosité est de 80%, la porosité étant définie comme le rapport (multiplié par 100) entre le volume des pores sur le volume total de la membrane (polymère + pores).

Le débit à l'eau (additionnée de 1% d'un agent tensio-actif) de cette membrane tramée est de 4,5 ml/h cm² mmHg.

Le taux de rejet au latex de cette membrane est de:
- 5 à 15% pour un latex calibré à 0,27 microns
- 65 à 80% pour un latex calibré à 0,4 microns

- 98 à 100% pour un latex calibré à 0,64 microns

Le capteur (13) est un capteur de marque NATIONAL SEMICONDUCTOR, référence LX 1 801 GB, dont la pression minimale affichée est réglée à 10 mm de mercure lors de la phase de prélèvement et la valeur maximale de pression est réglée à 80 mm de mercure (en valeur relative) lors de la phase de retour. Le capteur (19) est un capteur de même marque et de même référence que le capteur (13). Le capteur (19) est réglé à une pression relative maximale de 60 mm de mercure. Les récipients (17) de recueil du sang et (20) de recueil du plasma sont à la pression atmosphérique et sont placés sans dénivellation par rapport à l'appareil à membrane (2). Etant donné que la pression de sortie du sang du compartiment (3) amont est de 10 mm de mercure, du fait de la perte de charge dans la ligne 16b, la pression transmembranaire moyenne est égale à:

$$\frac{\text{à } 60 + 10}{2} = 35 \text{ mm de mercure}$$

Durant chaque phase de prélèvement le garrot est gonflé à 60 mm de mercure et le débit du sang citraté est de 60 ml/mm dans la ligne (5), entre l'aiguille (1) et la pompe (7). La vitesse de recirculation du sang dans la boucle (16, 16b) est de 170 ml/mm durant chaque phase de prélèvement.

Avec l'appareillage tel que précédemment défini, en se fixant un volume sang de 450 cm³ lors de la phase de prélèvement, un volume total de 600 cm³ de plasma à recueillir et un rapport volume solution anticoagulant/volume de sang de 1/16, pendant chaque phase de prélèvement, l'opération de plasmaphérèse a été terminée en 50 minutes après avoir effectué 5 phases de prélèvement et 5 phases de retour.

Le plasma recueilli est pratiquement acellulaire. Il ne contient aucune contamination en globules rouges et seulement 3000 plaquettes par mm³. La concentration protéique du plasma est de 57 g/l. L'appareillage selon l'invention a donc permis d'obtenir un rendement de séance de 20% avec un appareil à membrane dont le rendement intrinsèque n'est que de 11,5%.

À titre de comparaison une opération de plasmaphérèse réalisée dans les mêmes conditions de débit sang dans la ligne (5), soit 60 ml/mm, et de pression transmembranaire avec le même appareil (2) à membrane, mais sans recirculation du sang, c'est-à-dire en supprimant la ligne (16b) et en ajoutant un capteur de pression entre la pompe (7) et l'appareil à membrane (dans l'appareillage selon les figures 1 et 4), a nécessité 87 minutes pour recueillir 600 ml de plasma.

L'appareillage tel que précédemment décrit peut bien évidemment être utilisé sur les animaux (chien, cheval ...) notamment pour des opérations de plasmaphérèse.

De façon générale cet appareillage peut être utilisé chaque fois qu'il est souhaité de ne piquer un sujet (homme ou animal) qu'à un seul endroit avec une simple aiguille (n'ayant qu'un canal interne) et de faire passer le liquide prélevé (généralement du sang) au contact d'une membrane semi-perméable à une vitesse de circulation supérieure à celle du liquide prélevé, le liquide n'ayant pas traversé la membrane retournant au sujet par la même canalisation que celle servant au prélèvement.

Ainsi l'appareillage tel que précédemment décrit peut être utilisé pour d'autres applications que la plasmaphérèse de don. En fonction des caractéristiques de séparation des membranes semi-perméables utilisées, il sera possible de n'appauvrir le sang en circulation que de certaines de ses protéines ou que de certains éléments constitutifs du plasma. On peut aussi procéder à des séances d'hémofiltration moyennant par exemple la réinjection d'un liquide de substitution asservie à la quantité de liquide filtré recueilli dans le récipient (20).

Cet appareillage peut être également utilisé pour des opérations d'échange plasmatique, c'est-à-dire en réinjectant au patient, lors de la phase de retour, un plasma en quantité équivalente à celle soutirée, grâce à une pompe et une canalisation (non représentées) se raccordant par exemple sur la canalisation (5) entre le détecteur (12) de bulles et la pompe (7). Si le patient a un shunt artério-veineux il n'est pas nécessaire d'utiliser le garrot (21).

L'appareillage selon la présente invention peut également être utilisé pour le traitement du liquide d'ascite d'un patient, le garrot n'étant pas nécessaire dans cette application, de même que le dispositif (8) de distribution du produit anticoagulant et que le détecteur de bulles (12).

## Revendications

1. Appareillage utilisable dans le domaine médical, comprenant en combinaison:

– un dispositif (1) de prélèvement d'un liquide d'un sujet,

– un appareil (2) à membrane semi-perméable séparant ce liquide en une fraction ayant traversé la membrane et en une fraction n'ayant pas traversé la membrane, ledit appareil comportant un compartiment amont (3) et un compartiment aval (4) séparés par une membrane (14),

– une canalisation (5) reliant le dispositif (1) de prélèvement à une tubulure (6) du compartiment amont (3) de l'appareil (2) à membrane,

– une pompe (7) disposée sur cette canalisation (5),

– un capteur de pression (13) du liquide circulant dans la canalisation (5), ledit capteur étant situé entre le dispositif (1) de prélèvement et la pompe (7), ce capteur agissant sur la vitesse de rotation de la pompe (7) qui lui est asservie,

– un récipient (20) de recueil du liquide ayant traversé la membrane, ce récipient (20) étant en communication avec la sortie du compartiment aval (4) de l'appareil (2) à membrane,

– des moyens (19) permettant de s'assurer que la pression transmembranaire du liquide circulant au contact de la membrane (14) ne dépasse pas une valeur de consigne, caractérisé en ce qu'il comprend:

une canalisation (16) reliant une tubulure (15) du compartiment amont (3) de l'appareil (2) à membrane à un récipient (17) de recueil de la fraction du liquide n'ayant pas traversé la membrane,

– une canalisation (16b) reliant le récipient (17) à la canalisation (5), la jonction entre la canalisation (16b) et la canalisation (5) se faisant en un point A situé entre la pompe (7) et la tubulure (6) du compartiment amont (3) de l'appareil (2) à membrane semi-perméable,

– une pompe (18) disposée sur la boucle définie par la canalisation (16), la canalisation (16b) et la partie de la canalisation (5) comprise entre la jonction A et la tubulure (6) du compartiment amont (3) de l'appareil (2) à membrane, les moyens (19) agissant sur la vitesse de rotation de la pompe (18) qui leur est asservie,

– en ce que la pompe (7) est capable de tourner dans les deux sens et en ce que le dispositif (1) de prélèvement sert à la réinjection au sujet de la fraction n'ayant pas traversé la membrane, ledit dispositif étant constitué d'une seule aiguille.

2. Appareillage selon la revendication 1, caractérisé en ce qu'il comporte un garrot (21) et des moyens pour le gonfler et le dégonfler, ledit garrot étant gonflé, lors de la phase dite de prélèvement au cours de laquelle le liquide est prélevé du sujet, tandis qu'il est dégonflé lors de la phase dite de retour, au cours de laquelle la fraction du liquide n'ayant pas traversé la membrane (14) est restituée au sujet.

3. Appareillage selon l'une quelconque des revendications précédentes, caractérisé en ce qu'il comprend de plus des moyens (27) pour connaître la quantité de liquide prélevée au sujet lors de la phase de prélèvement et pour inverser le sens de rotation de la pompe (7) lorsqu'un volume prédéterminé est atteint dans le récipient (17).

4. Appareillage selon l'une quelconque des revendications précédentes, caractérisé en ce qu'il comprend des moyens permettant la vérification de la fin d'une phase de retour et assurant la commutation d'une phase de retour à une phase de prélèvement.

5. Appareillage selon l'une quelconque des revendications précédentes, caractérisé en ce qu'il comprend des moyens (30) permettant de connaître la quantité de liquide ayant passé dans le récipient (20) en relation avec le compartiment aval (4) de l'appareil (2) à membrane semi-perméable.

6. Appareillage selon l'une quelconque des revendications précédentes, caractérisé en ce qu'il comprend un piège à bulles (12).

7. Appareillage selon l'une quelconque des revendications précédentes, caractérisé en ce qu'il comprend un dispositif (8) pour introduire, lors de la phase de prélèvement, un produit anticoagulant dans la canalisation (5), à proximité du dispositif (1) de prélèvement.

8. Appareillage selon l'une quelconque des revendications précédentes, caractérisé en ce qu'il comprend une unité logique (22) de commande et de contrôle à laquelle sont reliés notamment les circuits électriques.

9. Appareillage selon l'une quelconque des revendications précédentes, caractérisé en ce qu'il comprend un clavier (28) sur lequel on peut choisir le volume de liquide que l'on veut faire arriver dans le récipient (17) lors de chaque phase de prélèvement et sur lequel on peut choisir le volume de liquide que l'on veut collecter dans le récipient (20) lors d'une séance complète.

10. Appareillage selon l'une quelconque des revendications précédentes, caractérisé en ce qu'il comprend une console sur laquelle sont regroupés les éléments constitutifs de l'appareillage.

## Patentansprüche

1. Anlage zur Verwendung im medizinischen Bereich, enthaltend in Kombination:

– eine Vorrichtung (1) zur Entnahme einer Flüssigkeit von einer Person,

– ein Gerät (2) mit semipermeabler Membran, das diese Flüssigkeit in einen Anteil trennt, der die Membran durchquert hat, und in einen Anteil trennt, der die Membran nicht durchquert hat, wobei das Gerät ein stromauf gelegenes Abteil (3) und ein stromab gelegenes Abteil (4) aufweist, die durch eine Membran (14) getrennt sind,

– eine Leitung (5), die die Entnahmevorrichtung (1) mit einem Anschlussstutzen (6) des stromauf gelegenen Abteils des Geräts (2) mit Membran verbindet,

– eine an dieser Leitung (5) gelegene Pumpe (7),

– einen Druckgeber (13) für die in der Leitung (5) zirkulierende Flüssigkeit, wobei dieser Geber auf die Drehzahl der ihm zugeordneten Pumpe (7) einwirkt,

– ein Gefäss (20) zur Aufnahme der Flüssigkeit, die die Membran durchquert hat, wobei dieses Gefäss mit dem Auslass des stromab gelegenen Abteils (4) des Geräts (2) mit Membran in Verbindung steht,

– Mittel (19), die sicherstellen, dass der Transmembrandruck der in Berührung mit der Membran (18) zirkulierenden Flüssigkeit einen Sollwert nicht übersteigt, dadurch gekennzeichnet, dass sie folgendes enhält:

– eine Leitung (16), die einen Anschlussstutzen (15) des stromauf gelegenen Abteils (13) des Geräts (2) mit Membran mit einem Gefäss (17) zur Aufnahme des Anteils der Flüssigkeit verbindet, die die Membran nicht durchquert hat,

– eine Leitung (16b), die das Gefäss (17) mit der Leitung (5) verbindet, wobei die Verbindungsstelle zwischen der Leitung (16b) und der Leitung (5) an einem Punkt (A) erfolgt, der zwischen der Pumpe (7) und der Leitung (6) des stromauf gelegenen Abteils des Geräts (2) mit semipermeabler Membran liegt,

– eine Pumpe (18) in dem Kreislauf, der durch die Leitung (16), die Leitung (16b) und den Teil der Leitung (5) zwischen der Verbindungsstelle (A) und dem Rohrstutzen (6) des stromauf gelegenen Abteils des Geräts (2) mit Membran definiert ist, wobei die Mittel (19) auf die Drehzahl der ihr zugeordneten Pumpe (18) einwirken, und

– dass die Pumpe (7) in beiden Richtungen rotieren kann und die Entnahmevorrichtung (1) dazu dient, der Person den Anteil wieder einzuspritzen, der die Membran nicht durchquert hat, wobei diese Vorrichtung aus einer einzigen Nadel besteht.

2. Anlage nach Anspruch 1, dadurch gekennzeichnet, dass sie eine Aderpresse (21) und Mittel zu deren Aufblasen und Lüften aufweist, wobei die Aderpresse während der Entnahmephase aufgeblasen wird, in deren Verlauf die Flüssigkeit der Person entnommen wird, während sie während der Rücklaufphase entlüftet wird, in deren Verlauf der Anteil der Flüssigkeit die die Membran nicht durchquert hat, der Person zurückgegeben wird.

3. Anlage nach einem der vorhergehenden Ansprüche, dadurch gekennzeichnet, dass sie Mittel (27) enthält zum Feststellen der bei der Person während der Entnahmephase entnommenen Flüssigkeit und zum Umkehren des Drehsinns der Pumpe (7), wenn ein gegebenes Volumen im Gefäss (17) erreicht ist.

4. Anlage nach einem der vorhergehenden Ansprüche, dadurch gekennzeichnet, dass sie Mittel enthält, die eine Kontrolle des Endes einer Rücklaufphase gestatten und die Umschaltung von einer Rücklaufphase in eine Entnahmephase gewährleisten.

5. Anlage nach einem der vorhergehenden Ansprüche, dadurch gekennzeichnet, dass sie Mittel (30) enthält, die das Feststellen der Flüssigkeitsmenge gestatten, die in das Gefäss (20) gelangt ist, in Beziehung zum stromab gelegenen Abteil (4) des Geräts (2) mit semipermeabler Membran.

6. Anlage nach einem der vorhergehenden Ansprüche, dadurch gekennzeichnet, dass sie eine Blasenfalle (12) enthält.

7. Anlage nach einem der vorhergehenden Ansprüche, dadurch gekennzeichnet, dass sie eine Vorrichtung (8) enthält, die während der Entnahmephase in Nähe der Entnahmevorrichtung (1) ein Antikoagulationsmittel in die Leitung (5) einführt.

8. Anlage nach einem der vorhergehenden Ansprüche, dadurch gekennzeichnet, dass sie eine logische Steuer- und Kontrolleinheit (22) enthält, mit der insbesondere die elektrischen Kreise verbunden sind.

9. Anlage nach einem der vorhergehenden Ansprüche, dadurch gekennzeichnet, dass sie eine Tastatur (28) enthält, auf der man das Flüssigkeitsvolumen wählen kann, das während jeder Entnahmephase in das Gefäss (17) gelangen soll, und auf der man das Flüssigkeitsvolumen wählen kann, das während einer vollständigen Sitzung im Gefäss (20) gesammelt werden soll.

10. Anlage nach einem der vorhergehenden Ansprüche, dadurch gekennzeichnet, dass sie eine Konsole enthält, auf der die die Anlage bildenden Elemente gruppiert sind.

**Claims**

1. Installation which can be used in the medical field, comprising in combination:

a device (1) for the withdrawal of a liquid from a subject,

an apparatus (2) containing a semi-permeable membrane separating this liquid into a fraction which has passed through the membrane and a fraction which has not passed through the membrane, the said apparatus containing an upstream compartment (3) and a downstream compartment (4) separated by a membrane (14),

a pipeline (5) connecting the withdrawal device (1) to a tubulure (6) from the upstream compartment (3) of the membrane-containing apparatus (2),

a pump (7) arranged on this pipeline (5),

a pressure sensor (13) of the liquid circulating in the pipeline (5), the said sensor being located between the withdrawal device (1) and the pump (7), this sensor acting on the rotation speed of the pump (7) which is under its control,

a container (20) for collecting the liquid which has passed through the membrane, this container (20) being in communication with the outlet of the downstream compartment (4) of the membrane-containing apparatus (2),

means (19) which make it possible to ensure that the pressure, across the membrane, of the liquid circulating in contact with the membrane (14) does not exceed a predetermined value, characterized in that it comprises:

a pipeline (16) connecting a tubulure (15) from the upstream compartment (3) of the membrane-containing apparatus (2) to a container (17) for collecting the fraction of the liquid which has not passed through the membrane,

a pipeline (16b) connecting the container (17) to the pipeline (5), the junction between the pipeline (16b) and the pipeline (5) being made at a point A located between the pump (7) and the tubulure (6) from the upstream compartment (3) of the apparatus (2) containing a semi-permeable membrane,

a pumpe (18) arranged on the loop defined by the pipeline (16), the pipeline (16b) and the part of the pipeline (5) included between the junction A and the tubulure (6) from the upstream compartment (3) of the membrane-containing apparatus (2), the means (19) acting on the rotation speed of the pump (18) which is under their control,

in that the pump (7) is capable of rotating in both directions and in that the withdrawal device (1) serves for reinjecting to the subject the fraction which has not passed through the membrane, the said device consisting of a single needle.

2. Installation according to claim 1, characterized in that it comprises a tourniquet (21) and means for inflating and deflating it, the said tourniquet being inflated during the phase referred to as the withdrawal phase, during which the liquid is withdrawn from the subject, whereas it is deflated during the phase referred to as the return phase, during which the fraction of the liquid which has not passed through the membrane (14) is restored to the subject.

3. Installation according to either one of the preceding claims, characterized in that it additionally comprises means (27) for determining the quantity of liquid withdrawn from the subject during the withdrawal phase and for reversing the direction of rotation of the pump (7) when a predetermined volume is reached in the container (17).

4. Installation according to any one of the preceding claims, characterized in that it comprises means which enable the end of a return phase to be verified and which ensure the change from a return phase to a withdrawal phase.

5. Installation according to any one of the preceding claims, characterized in that it comprises means (30) which enable the quantity of liquid which has passed into the recipient (20) associated with the downstream compartment (4) of the semi-permeable membrane-containing apparatus (2) to be determined.

6. Installation according to any one of the preceding claims, characterized in that it comprises a bubble trap (12).

7. Installation according to any one of the preceding claims, characterized in that it comprises a device (8) for introducing, during the withdrawal phase, an anti-coagulant into the pipeline (5), close to the withdrawal device (1).

8. Installation according to any one of the preceding claims, characterized in that it comprises a logic unit (22) for controlling and monitoring, to which especially the electrical circuits are connected.

9. Installation according to any one of the preceding claims, characterized in that it comprises a keyboard (28) on which the volume of liquid which it is desired should be brought to the container (17) during each withdrawal phase may be chosen and on which the volume of liquid which it is desired to collect in the container (20) during a complete session may be chosen.

10. Installation according to any one of the preceding claims, characterized in that it comprises a console on which the constituent components of the installation are gathered.

Fig. 1

0 131 529

2/4

Fig. 2

V max.

V₀

17

9

8

10

11

1

5

13

12

7

16

16b

A

19

18

6

4

14

3

15

2

20

21

fig. 3

0 131 529

fig. 4